# EUROPEAN PATENT SPECIFICATION

(11) **EP 3 519 588 B1**
(45) Date of publication and mention of the grant of the patent: **02.12.2020**
(21) Application number: 17784203.6
(22) Date of filing: 27.09.2017
(51) Int. Cl.: C12Q 1/6841

(54) **METHOD FOR LABELING OLIGONUCLEOTIDE PROBES**
VERFAHREN ZUR MARKIERUNG VON OLIGONUKLEOTIDSONDEN
PROCEDE DE MARQUAGE D'OLIGONUCLEOTIDES

(30) Priority: 27.09.2016 EP 16190862
(43) Date of publication of application: 07.08.2019
(73) Proprietor: Deutsches Krebsforschungszentrum Stiftung des Öffentlichen Rechts, 69120 Heidelberg (DE)
(72) Inventor: CHENG, Yong-Sheng, 69198 Schriesheim (DE); LIU, Hai-Kun, 69469 Weinheim (DE)
(74) Representative: Kuttenkeuler, David
(86) International application number: PCT/EP2017/074505
(87) International publication number: WO 2018/060249

(56) References cited:
- WO-A1-2012/148477
- YU LIU ET AL: "Specific labeling: An effective tool to explore the RNA world", BIOESSAYS, vol. 38, no. 2, 14 December 2015 (2015-12-14), pages 192-200, XP055354654, GB ISSN: 0265-9247, DOI: 10.1002/bies.201500119
- B. MORITZ ET AL: "Simple methods for the 3' biotinylation of RNA", RNA, vol. 20, no. 3, 21 January 2014 (2014-01-21), pages 421-427, XP055354650, US ISSN: 1355-8382, DOI: 10.1261/rna.042986.113
- NILSEN TIMOTHY W: "Site-specific labeling of RNA", COLD SPRING HARBOR PROTOCOLS, COLD SPRING HARBOR LABORATORY PRESS, US, vol. 2013, no. 3, 1 March 2013 (2013-03-01), pages 269-275, XP009183741, ISSN: 1559-6095, DOI: 10.1101/PDB.PROT072918
- F. BEHNAM ET AL: "A Straightforward DOPE (Double Labeling of Oligonucleotide Probes)-FISH (Fluorescence In Situ Hybridization) Method for Simultaneous Multicolor Detection of Six Microbial Populations", APPLIED AND ENVIRONMENTAL MICROBIOLOGY, vol. 78, no. 15, 11 May 2012 (2012-05-11), pages 5138-5142, XP055355298, US ISSN: 0099-2240, DOI: 10.1128/AEM.00977-12

## Description

The present invention provides a novel method for labelling nucleic acid probes according to the appended set of claims. The method uses a ligase catalysed reaction to connect a nucleic acid probe with pre-prepared nucleic acid label carrier molecules under the presence of a stabilizing complementary splint oligonucleotide. The method allows for an easy, cheap and fast labelling of multiple probes with multiple different labels. In this way, the costs and effort for the generation of single molecule Fluorescent *In Situ* Hybridization (smFISH) assays was significantly reduced. The invention further provides methods for the generation of FISH libraries and labelling kits comprising the novel tools of the invention.

WO 2012/148477 discloses splint-assisted, ligation-based labelling of nucleic acids.

### DESCRIPTION

Since the invention of in situ hybridization (ISH) by Joseph G. Gall and Mary-Lou Pardue more than 40 years ago, this powerful technique has tremendously transformed basic research (i.e. gene expression, etc.) and diagnostics for biomarker detection. Only through ISH, researchers were able to study gene expression and diagnose biomarker level with spatio-information. Nowadays, ISH and its derivatives have become the working horse in a variety of fields, including histology, single cell biology, etc. There were even omics-scale efforts trying to have a complete gene expression atlas like Allen Brain Atlas based on ISH. With the advances in fluorophore chemistry and microscopy hardware, Fluorescence *in situ* hybridization (FISH) has gained more attentions due to its superior low background and nm-scale spatio-localized signal (vs diffusive signal generated in enzymatic reaction from ISH), and could serve as good alternative for ISH, especially in future applications in the precision medicine where requiring digital and spatiotemporal quantification of biomarkers.

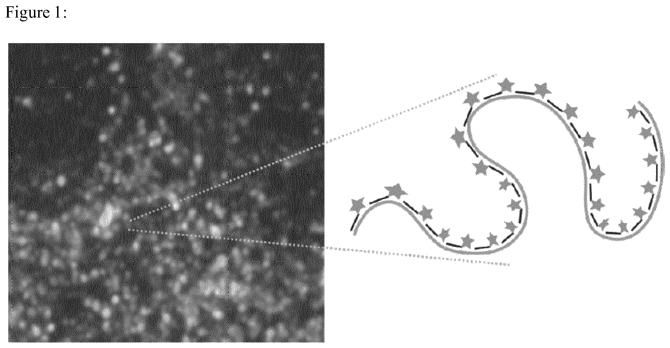

Since 90s, FISH has been innovatively used by Robert Singer and colleagues to image individual mRNA transcripts in situ at single molecular level (smFISH) from then gene expression or biomarker detection could be done in a digital and single molecular fashion (Figure 1). However the first probe library was made from five gene-specific different custom-synthesized penta-fluorophore labelled oligos, which is still very expen- sive nowadays and make this powerful invention to be not applicable for every gene. From 2008, Arjun and colleagues invented a pooled single fluorophore labelling method based on classical EDC(N-(3-Dimethylaminopropyl)-N'-ethylcarbodiimide hydrochloride) conjugation chemistry, which greatly downsizes the cost of probe library. And this novel labelling is now licensed by Biosearch Technologies Inc. so research and clinical users could use this technique at price of ∼760 euro for 100-400 reactions. This exceptionally high cost for using the commercial FISH probe library is a fundamental limitation of smFISH, as well as its low throughput, typically smFISH approach allow probing only a few genes at a time. This low throughput is due to a lack of distinguishable probes with which to label cells and the cost of producing large amounts of labeled probe required for high efficient staining. Thus, improvements in smFISH probe generation and improving detection efficiency is necessary. Before the present invention is described, it is to be understood that this invention is not limited to particular embodiments described, and as such may, of course, vary. It is also to be understood that the terminology used herein is for the purpose of describing particular embodiments only, and is not intended to be limiting, since the scope of the present invention will be limited only by the appended claims.

The above problem is solved by a method for producing a labelled or otherwise modified, oligonucleotide probe, the method comprising the steps of:
a. Providing a probe-sequence-oligonucleotide comprising a nucleotide sequence complementary to a target nucleic acid,
b. Providing a label-carrier-oligonucleotide comprising at least one labeling moiety, or other functional moiety, wherein the label-carrier-oligonucleotide has a predetermined nucleotide sequence,
c. Providing a complementary-splint-oligonucleotide, comprising (i) a reverse complementary region having a sequence that is reverse complementary to a sequence of the label-carrier-oligonucleotide and (ii) a random sequence region comprising a random nucleotide sequence;
d. Bringing into contact under hybridizing conditions the probe-sequence-oligonucleotide, label-carrier-oligonucleotide and the complementary-splint-oligonucleotide, to form a complex, wherein, in the complex, a free (unblocked) -OH group is in close spatial proximity to a free (unblocked) phosphate group,
e. Reacting the complex to form a covalent bond between the 3'-OH group and the 5'-phosphate group using a ligase under ligating conditions, to form the labeled, or otherwise modified, oligonucleotide probe ("ligation product"),
f. Optionally, removing the complementary-splint-oligonucleotide.

In one embodiment of the invention the label-carrier-oligonucleotide is ligated to the 3 prime end of the probe-sequence-oligonucleotide. In this embodiment, the method of the invention comprises the steps of:
a. Providing a probe-sequence-oligonucleotide comprising a nucleotide sequence complementary to a target nucleic acid and having a free (unblocked) -OH group at the 3' end,
b. Providing a label-carrier-oligonucleotide comprising at least one labeling moiety, or other functional moiety, wherein the label-carrier-oligonucleotide has a predetermined nucleotide sequence, and a free (unblocked) phosphate group at the 5' end,
c. Providing a complementary-splint-oligonucleotide, comprising a sequence having in 5' to 3'-direction (i) a reverse complementary region comprising a sequence that is reverse complementary to a sequence of the label-carrier-oligonucleotide; and (ii) a random sequence region comprising a random nucleotide sequence, optionally wherein the complementary-splint-oligonucleotide comprises a 3'-end blocking group,
d. Bringing into contact under hybridizing conditions the probe-sequence-oligonucleotide, label-carrier-oligonucleotide and the complementary-splint-oligonucleotide to form a complex, wherein in the complex the free (unblocked) -OH group at the 3' end of the probe-sequence-oligonucleotide is in close spatial proximity to the free (unblocked) phosphate group at the 5' end of the label-carrier-oligonucleotide,
e. Reacting the complex to form a covalent bond between the 3 '-end of the probe-sequence-oligonucleotide and the 5'-end of the label-carrier-oligonucleotide using a ligase under ligating conditions, to form the labeled, or otherwise modified, oligonucleotide probe,
f. Optionally, removing the complementary-splint-oligonucleotide.

In one further embodiment of the invention the label-carrier-oligonucleotide is ligated to the 5 prime end of the probe-sequence-oligonucleotide. In this embodiment, the method of the invention comprises the steps of:
a. Providing a probe-sequence-oligonucleotide comprising a nucleotide sequence complementary to a target nucleic acid, having a free (unblocked) -phosphate group at the 5' end,
b. Providing a label-carrier-oligonucleotide comprising at least one labeling moiety, or other functional moiety, wherein the label-carrier-oligonucleotide has a predetermined nucleotide sequence, and a free (unblocked) -OH group at the 3' end,
c. Providing a complementary-splint-oligonucleotide, comprising a sequence having in 3' to 5'-direction (i) a reverse complementary region comprising a sequence that is reverse complementary to the sequence of the label-carrier-oligonucleotide; and (ii) a random sequence region comprising a random nucleotide sequence, and wherein the complementary-splint-oligonucleotide comprises a 3'-end blocking group,
d. Bringing into contact under hybridizing conditions the probe-sequence-oligonucleotide, label-carrier-oligonucleotide and the complementary-splint-oligonucleotide to form a complex, wherein in the complex the free (unblocked) -OH group at the 3' end of the label-carrier-oligonucleotide is in close spatial proximity to the free (unblocked) phosphate group at the 5' end of the probe-sequence-oligonucleotide,
e. Forming a covalent bond between the 5'-end of the probe-sequence-oligonucleotide and the 3'-end of the label-carrier-oligonucleotide using a ligase under ligating conditions, to form the labeled, or otherwise modified, oligonucleotide probe,
f. Optionally, removing the complementary-splint-oligonucleotide.

The methods and compounds of the invention allow for a quick, cheap and easy labelling of nucleic acid probes. The idea of the invention is to provide a system that allows the practitioner to label any given oligonucleotide probe (probe-sequence-oligonucleotide) on the shelf without the need to attach the label-moiety to the individual probe sequence - which is expensive and therefore impairs the generation of libraries of many labelled individual probes. To this end, the invention now provides a system comprising one or more pre-prepared label-carrying-oligoncleotide(s), possibly a selection of label-carrying-oligoncleotide(s) each having different labels or other modifications, or combinations thereof, to select from depending on the probe usage. Using a complementary-splint-oligonucleotide according to the invention, these label carriers are simply ligated to the probe oligonucleotide to obtain the labelled probe product. Since it is possible to label large quantities of identical label-carrying-oligoncleotide(s) which then can be used in the easy and cheap ligation reaction, costs are significantly reduced.

In context of the invention, when referring to a specific oligonucleotide, or probe, or to other expressions describing nucleic acids, the person of skill will recognize that the expression refers not to single molecules, but to a single species of molecules. In practical application in the laboratory, the person of skill uses preparations of a multitude of molecules of a species of, for example, one oligonucleotide sequence. In such a population of molecules usually all nucleotides are identical, except where the oligonucleotides were produced to contain a random sequence region, for example during oligonucleotide synthesis. This can be achieved by polymerising the oligonucleotide with an equal mixture of more than one type of nucleotide, which then are coupled by chance. In this event the species of oligonucleotides in the mixture contains a "degenerated" sequence.

The term "nucleoside" and "nucleotide" are intended to include those moieties that contain not only the known purine and pyrimidine bases, but also other heterocyclic bases that have been modified. Such modifications include methylated purines or pyrimidines, acylated purines or pyrimidines, alkylated riboses or other heterocycles. In addition, the term "nucleotide" includes those moieties that contain hapten or fluorescent labels and may contain not only conventional ribose and deoxyribose sugars, but other sugars as well. Modified nucleosides or nucleotides also include modifications on the sugar moiety, e.g., wherein one or more of the hydroxyl groups are replaced with halogen atoms or aliphatic groups, are functionalized as ethers, amines, or the like.

The term "nucleic acid" refers to a polymer of any length, e.g., greater than about 2 bases, greater than about 10 bases, greater than about 100 bases, greater than about 500 bases, greater than 1000 bases, up to about 10,000 or more (e.g., 100,000,000 or more) bases composed of nucleotides, e.g., deoxyribonucleotides or ribonucleotides, and may be produced enzymatically or synthetically which can hybridize with naturally occurring nucleic acids in a sequence specific manner analogous to that of two naturally occurring nucleic acids, e.g., can participate in Watson-Crick base pairing interactions. Naturally-occurring nucleotides include guanine, cytosine, adenine and thymine/uracil (G, C, A and T/U, respectively).

The term "oligonucleotide" as used herein denotes a single stranded multimer of nucleotide of from about 2 to about 500 nucleotides. Oligonucleotides may be synthetic or may be made enzymatically. Oligonucleotides may contain ribonucleotide monomers (i.e., may be oligoribonucleotides), deoxyribonucleotide monomers or a combination of the two. Oligonucleotides may be 10 to 20, 11 to 30, 31 to 40, 41 to 50, 51-60, 61 to 70, 71 to 80, 80 to 100, 100 to 150, 150 to 200 or 200-250 or up to 500 nucleotides in length.

The term "hybridization" refers to the specific binding of a nucleic acid to a complementary nucleic acid via Watson-Crick base pairing. Accordingly, the term "in situ hybridization" refers to specific binding of a nucleic acid to a complementary nucleic acid inside a cell or in an intact chromosome. The terms "hybridizing" and "binding", with respect to nucleic acids, are used interchangeably.

The term "hybridizing conditions" is intended to mean those conditions of time, temperature, and pH, and the necessary amounts and concentrations of reactants and reagents, sufficient to allow at least a portion of complementary sequences to anneal with each other. As is well known in the art, the time, temperature, and pH conditions required to accomplish hybridization depend on the size of the oligonucleotide molecules to be hybridized, the degree of complementarity between the oligonucleotides to be hybridized, and the presence of other materials in the hybridization reaction admixture, salts etc.. The actual conditions necessary for each hybridization step are well known in the art or can be determined without undue experimentation.

The term "in situ hybridization conditions" as used herein refers to conditions that allow hybridization of a nucleic acid to a complementary nucleic acid, e.g., a sequence of nucleotides in a RNA or DNA molecule and a complementary oligonucleotide, in a cell. Suitable in situ hybridization conditions may include both hybridization conditions and optional wash conditions, which conditions include temperature, concentration of denaturing reagents, salts, incubation time, etc. Such conditions are known in the art.

The phrase "random nucleotide sequence" refers to a varied sequence of nucleotides that when combined with other random nucleotide sequences in a population of polynucleotides represent all or substantially all possible combinations of nucleotides for a given length of nucleotides. For example, because of the four possible nucleotides present at any given position, a sequence of two random nucleotides in length has 16 possible combinations, a sequence of three random nucleotides in length has 64 possible combinations, or a sequence of four random nucleotides in length has 265 possible combination. Accordingly, when used in reference to the methods of the invention, a random nucleotide sequence has the potential to hybridize to any target polynucleotide in the sample. In preferred embodiments the random sequence comprises approximately 25% of each nucleotide type (adenine, thymine/uracil, cytosine and guanine). Such a sequence stretch is also referred to as being "degenerated".

In some embodiments of the invention, the probe-sequence-oligonucleotide, the label-carrier-oligonucleotide and the complementary-splint-oligonucleotide are single stranded nucleic acid molecules, preferably RNA and/or DNA. Furthermore, all nucleic acid molecules may contain un-natural or modified nucleic acid residues, such as O-methly modified residues. However, specifically preferred are DNA oligonucleotides which are easier and cheaper to synthesize.

It is in some embodiments preferred that the probe-sequence-oligonucleotide does not comprise a modification of the 3' terminal OH group and/or 5' terminal phosphate group of the polynucleotide, in particular it is preferable that the probe-sequence-oligonucleotide does not comprise a terminal amine group. In context of the invention it is likely that the probe-sequence-oligonucleotide will be chemically synthesized according to state of the art oligonucleotide synthesis procedures. Such procedures often include the use of masking or blocking groups, which preferably are all removed before applying the probe-sequence-oligonucleotide to the method of the invention. Also, synthesis of oligonucleotides often results in a 5 prime terminal OH group. In some embodiments where the label-carrier-oligonucleotide is ligated to the 5 prime end of the probe-sequence-oligonucleotide, it is necessary to attach a terminal 5 prime phosphate group to allow for the ligation reaction.

In some embodiments it is preferable that the label-carrier-oligonucleotide comprises two or more, preferably three, four or five, labelling moieties, or other functional moieties. Most preferably, the multiple labels provide for a unique label combination. In this embodiment the label-carrier-oligonucleotide may be colour barcoded by the unique combination of multiple colour labels. This embodiment allows a simultaneous probing of multiple target sequences such as mRNA species in a cell.

The label-carrier-oligonucleotide may be of any length suitable for ligation with the probe sequence oligonucleotide. Some preferred label-carrier-oligonucleotide of the invention are 3 to 200 nucleotides in length, preferably 3 to 100 nucleotides, more preferably 3 to 30, or 5 to 20.The sequence of the label-carrier-oligonucleotide is not important or detrimental for the labelling method of the invention. However, it may be preferred that the sequence is selected based on the later usage of the labelled oligonucleotide probe, for example to avoid a binding to a non-target sequence via the label-carrier-oligonucleotide sequence, or to allow the attachment of multiple label molecules such as fluorophores.

The label-carrier-oligonucleotide in one preferred embodiment comprises a 5 prime (free) phosphate group to allow for an enzyme catalysed ligation with the probe-sequence-oligonucleotide. In another embodiment the label-carrier-oligonucleotide comprises a 3 prime end terminal OH group.

The term "splint oligonucleotide" generally refers to an oligonucleotide that has a first sequence of nucleotides complimentary to a region of the first oligonucleotide adjacent to the terminal end, and a second sequence of nucleotides complimentary to a region of the second oligonucleotide adjacent to the terminal end. The splint oligonucleotide is capable of binding both the first and second oligonucleotides to produce a complex, and thus to bring the terminals of the first and second oligonucleotides into spatial proximity. By bringing the terminals of the first and second oligonucleotide into proximity, the splint oligonucleotide increases the chance of ligation between the first and second oligonucleotide. The first and second oligonucleotides are in the present context the probe-sequence-oligonucleotide and the label carrier oligonucleotide. Since for the present application the sequence of the probe-sequence oligonucleotide is variable, complementarity of the splint is provided by using a random (degenerated) sequence as described elsewhere in the present disclosure. Therefore, a complementary-splint-oligonucleotide of the invention comprises (i) a reverse complementary region and (ii) a random sequence region, and region (i) mediates hybridization to the label-carrier-oligonucleotide, and region (ii) mediates hybridization to the probe-sequence-oligonucleotide. In some preferred embodiments there are no nucleotides between (i) and (ii), meaning that both regions are directly adjacent to each other. The reverse complementary region is preferably located in the complementary-splint-oligonucleotide such, that upon binding/hybridization of the label-carrier-oligonucleotide and the complementary-splint-oligonucleotide, the reverse complementary region is positioned directly adjacent to the free end of the label-carrier-oligonucleotide that is to be ligated with the probe-sequence-oligonucleotide (as illustrated in the appended figures). Thereby, the complementary-splint-oligonucleotide of the invention serves as a "splint" to position the label-carrier-oligonucleotide and the probe-sequence-nucleotide directly next to each other, so their respective 3 prime and 5 prime ends can be ligated.

The complementary-splint-oligonucleotide comprises preferably a reverse complementary region having at least 3, preferably 4, 5, 6, 7, 8 or more nucleic acids. In some embodiments the reverse complementary region consists of a sequence reverse complementary to the sequence of the label-carrier-oligonucleotide. Even more preferably the sequence of the complementary-splint-oligonucleotide of the invention consists of the random sequence region and the reverse complementary region. The degree of complementarity between the reverse complementary region of the complementary-splint-oligonucleotide and the label-carrier-oligonucleotide is selected to allow for the stable hybridization of the two molecules under conditions suitable for performing a ligation. In this context the degree of reverse complementarity is preferably at least 90%, most preferably 95% or 100%.

Preferred label-carrier-oligonucleotide and/or complementary-splint-oligonucleotide sequences of the invention are provided in SEQ ID NO: 1, 2 and 35 (the latter for a dual label carrying label-carrier-oligonucleotide). It is understood that the sequences are preferred without any restriction to the position or nature of the labels that were actually used to proof the concept of the invention.

In some embodiments the method may further comprise a step of purifying the ligated product of the probe-sequence-oligonucleotide and label-carrier-oligonucleotide. The ligation product forms the labelled, or otherwise modified, oligonucleotide probe, and therefore the product of the inventive process. Purification may be done by any means known to the skilled artisan for purifying nucleic acid probes, and includes but is not limited to, purification via gel electrophoreses, such as PAGE.

In some embodiments the method may further comprise a step of providing an adaptor oligonucleotide comprising a sequence complementary to a sequence of the label-carrier-oligonucleotide, and bringing into contact under hybridizing conditions the ligated product of the probe-sequence-oligonucleotide and label-carrier-oligonucleotide with the adaptor oligonucleotide, to form a stabilized oligonucleotide probe. This embodiment is preferable when the label-carrier-oligonucleotide comprises multiple labelling moieties. Because multiple label moieties may in a single stranded nucleic acid probe quench each other, the probe can be stabilized by forming a double stranded region at the labelled end via hybridizing the adaptor oligonucleotide to the label-carrier-oligonucleotide region of the ligation product.

The term "ligase" as used herein refers to an enzyme that is commonly used to join polynucleotides together or to join the ends of a single polynucleotide. A ligase of the invention is preferably selected from ATP-dependent double-strand polynucleotide ligases, NAD+-dependent double-strand DNA or RNA ligases, and single-strand polynucleotide ligases, and are preferably selected from bacterial ligases, such as E. coli DNA ligase, Taq DNA ligase, Ampligase® thermostable DNA ligase, phage ligases, such as T3 DNA ligase, T4 DNA ligase, T7 DNA ligase and mutants thereof, including fusion ligases containing a DNA-binding domain and a ligase, such as Sso7-T3 DNA ligase, Sso7-T4 DNA ligase, Sso7-T7 DNA ligase, Sso7-Taq DNA ligase, Sso7-E. coli DNA ligase, Sso7-Ampligase, DNA ligase Sso7, T4 RNA ligase 1, T4 RNA ligase 2, and T4 truncated and mutated (K227Q) RNA ligase. Most preferably is the use of T4 DNA ligase, because this enzyme is robust, cheap and efficient.

The term "target" refers to a biological entity that can be spatially separated, hybridized to a probe, and visualized. Cells, individual chromosomes, and material deposited in an array are examples of targets. In context of the invention the target nucleic acid is a target single stranded nucleic acid, that comprises a sequence having at least one, preferably multiple, binding regions for nucleic acid probes as produced by the method of the present invention. In preferred embodiments the target nucleic acid is a messenger RNA (mRNA).

A label in context of the invention is a detectable moiety that may produce a signal directly or indirectly. One example of a detectable moiety that produces a signal directly is a fluorescent molecule. Detectable moieties that produce a signal indirectly include moieties that produce a signal upon exposure to detection reagents such as substrates or antibodies, etc. A detectable moiety that produces a signal directly can optionally be detected by indirect means such as by using a labeled antibody that binds to the moiety. In certain cases, a signal may be of a particular wavelength that is detectable by a photodetector, e.g., a light microscope, a spectrophotometer, a fluorescent microscope, a fluorescent sample reader, or a florescence activated cell sorter, etc. A labeling moiety in context of the invention may be any moiety that allows for detection of the presence or absence of the moiety. Suitable labels include fluorescent dyes that include xanthene dyes, e.g. fluorescein and rhodamine dyes, such as fluorescein isothiocyanate (FITC), 6-carboxyfluorescein (commonly known by the abbreviations FAM and F),6-carboxy-2',4',7',4,7-hexachlorofluorescein (HEX), 6-carboxy-4',5'-dichloro-2',7'-dimethoxyfluorescein (JOE or J), N,N,N',N'-tetramethyl-6-carboxyrhodamine (TAMRA or T), 6-carboxy-X-rhodamine (ROX or R), 5-carboxyrhodamine-6G (R6G⁵ or G⁵), 6-carboxyrhodamine-6G (R6G⁶ or G⁶), and rhodamine 110; cyanine dyes, e.g. Cy3, Cy5 and Cy7 dyes; coumarins, e.g umbelliferone; benzimide dyes, e.g. Hoechst 33258; phenanthridine dyes, e.g. Texas Red; ethidium dyes; acridine dyes; carbazole dyes; phenoxazine dyes; porphyrin dyes; polymethine dyes, e.g. cyanine dyes such as Cy3, Cy5, etc; BODIPY dyes and quinoline dyes. Specific fluorophores of interest that are commonly used in some applications include: pyrene, coumarin, diethylaminocoumarin, FAM, fluorescein chlorotriazinyl, R110, eosin, JOE, R6G, tetramethylrhodamine, TAMRA, lissamine, ROX, napthofluorescein, Texas red, napthofluorescein, Cy3, and Cy5, etc. Suitable distinguishable fluorescent label pairs useful in the subject methods include Cy-3 and Cy-5 (Amersham Inc., Piscataway, N.J.), Quasar 570 and Quasar 670 (Biosearch Technology, Novato Calif.), preferably the following four Alexa dyes: Alexa fluor 488, Alexa fluor 555, Alexa fluor 594 and Alexa fluor 647 (Molecular Probes, Eugene, Oreg.); BODIPY V-1002 and BODIPY V1005 (Molecular Probes, Eugene, Oreg.), POPO-3 and TOTO-3 (Molecular Probes, Eugene, Oreg.), and POPRO3 TOPRO3 (Molecular Probes, Eugene, Oreg.). Further suitable distinguishable detectable labels may be found in Kricka et al. (Ann Clin Biochem. 39:114-29, 2002).

The phrase "distinguishable labels" or "different-color labels" or any grammatical equivalent thereof refers to labels can be independently detected and measured, even when the labels are mixed. In other words, the amounts of label present (e.g., the amount of fluorescence) for each of the labels are separately determinable, even when the labels are co-located (e.g., in the same tube or in the same duplex molecule or in the same cell). The above labels may be used as distinguishable labels. Some preferred distinguishable fluorescent label pairs include Cy-3 and Cy-5 (Amersham Inc., Piscataway, N.J.), Quasar 570 and Quasar 670 (Biosearch Technology, Novato Calif.), Alexafluor555 and Alexafluor647 (Molecular Probes, Eugene, Oreg.), BODIPY V-1002 and BODIPY V1005 (Molecular Probes, Eugene, Oreg.), POPO-3 and TOTO-3 (Molecular Probes, Eugene, Oreg.), and POPRO3 and TOPRO3 (Molecular Probes, Eugene, Oreg.), and preferably FAM and ATTO550. Further suitable distinguishable detectable labels may be found in Kricka et al. (Ann Clin Biochem. 39:114-29, 2002).

The term "predetermined" refers to something that is known before use.

Preferably the probe-sequence-oligonucleotide to be used in the methods of the invention has a length of between 20 to 300 nucleotides. Depending on which kind of target nucleic acid is to be detected by the probe produced according to the invention, the length of the probe is selected. Although for the application of smFISH probes the length of the probes is selected to be below 500 bases, other applications may require the use of longer nucleic acid probes. Since the length of the probe sequence is not important for the labelling process, the present invention shall not be limited to any specific lengths. Preferred is however a probe sequence for use in smFISH.

In context of the invention the random sequence region of the complementary-splint-oligonucleotide comprises a degenerated sequence, which means a randomly generated overhang that is intended to bind un-specifically to the sequence of the probe-sequence-oligonucleotide. The random sequence region of the complementary-splint-oligonucleotide may consist of 2 to 10, preferably 2 to 8, more preferably 2 to 6, and most preferably 4 nucleotides.

In some preferred embodiments of the invention the 3-prime end of the complementary-splint-oligonucleotide is blocked with an amine (NH₂) group.

The problem is furthermore solved by a method for generating a single molecule Fluorescent *In Situ* Hybridization (smFISH) probe library, comprising producing at least two fluorescent labeled oligonucleotide probes according to a labelling method of the above disclosed invention, wherein the at least two fluorescent oligonucleotide probes are capable of binding to one target nucleic acid.

Some embodiments of the invention pertain to the above library generation method where the at least two fluorescent labeled oligonucleotide probes are capable of binding the one target nucleic acid at different, preferably non-overlapping, locations (sequences). The at least two fluorescent labeled oligonucleotide probes in a library of the invention are at least 10, preferably at least 30, more preferably 30 to 150, and most preferably about 100 fluorescent labeled oligonucleotide probes, and wherein each of said fluorescent labeled oligonucleotide probes is capable of binding to the one target nucleic acid, preferably at non-overlapping positions.

In some preferred embodiments of this aspect, at least two of the at least two fluorescent labeled oligonucleotide probes in the library are labeled with multiple label moieties, however, preferably wherein each labeled oligonucleotide probe in the library comprises an identical label, or identical label combination. A library of nucleic acid probes in context of the invention shall denote a set of probes for probing and detecting one nucleic acid molecule, for example one mRNA in a smFISH approach.

In context of the invention the cell is preferably a fixed and permeabilized cell.

In this aspect the "oligonucleotide probe(s)" are prepared preferably according to a labeling method of the invention as disclosed herein above.

The number of probe binding regions in the target mRNAs and probes may in some embodiments be 40 to 60. In other embodiments the at least 30 probes have target-complementary sequences that are 7-40 nucleotides in length, most preferably that are 15-30 nucleotides in length.

Each probe may be added to the cell in a concentration of 0.2- 10 nanograms per microliter.

The fixed cells are preferably prepared by formaldehyde fixation.

Detection includes preferably imaging with a wide-field fluorescence microscope.

Furthermore provided is a labelling kit for labelling an oligonucleotide probe, the kit comprising a label-carrier-oligonucleotide comprising at least one labeling moiety, or other functional moiety, wherein the label-carrier-oligonucleotide has a predetermined nucleotide sequence; a complementary-splint-oligonucleotide, comprising (i) a reverse complementary region having a sequence that is reverse complementary to a sequence of the label-carrier-oligonucleotide and (ii) a random sequence region comprising a degenerated nucleotide sequence. The specific descriptions for the components of the method of the invention equally apply to the respective components of the labelling kit of the invention.

In certain embodiments the labelling kit of the invention may comprise a multitude of differently labelled label-carrier-oligonucleotides. In this embodiment the labelling kit provides at least two label-carrier-oligonucleotides coupled to different-color label moieties. In most preferred embodiments the labeling kit comprises multiple label-carrier-oligonucleotides, and to each of the label-carrier-oligonucleotides a multitude (at least two) label moieties are coupled. In this labelling kit each label-carrier-oligonucleotide comprises a different label combination, such as individual color combinations. These individual color combinations may serve as label barcodes for the label-carrier-oligonucleotides to generate oligonucleotide probes for multiple target sequences. This labeling kit preferably is used in an smFISH approach to probe multiple mRNA simultaneously.

The labeling kit of the invention may further comprise a ligase, optionally together with buffers or reagents for its use. Preferable ligases are described herein above.

In another embodiment of the invention the labelling kit may further comprise an adaptor oligonucleotide as described herein above.

The labeling kit of the invention may further comprise instructions for its use.

The present invention will now be further described in the following examples with reference to the accompanying figures and sequences, nevertheless, without being limited thereto. In the Figures:
- **Figure 1:**: **Molecular architecture of smFISH.** Multiple single or multiple fluorescently labelled gene-specific hybridization probes are bound to target mRNA in situ. Due to the local concentrated fluorophores, individual mRNA can be visualized on light microscopy as single dot within the diffraction limit (200 nm in size).
- **Figure 2:**: **T4 DNA ligase mediated fluorescent labelling of pool smFISH oligos.** Unlabelled smFISH probes are bridged together with a common fluorescent oligo by spint DNA with random quadmer at 5' and an additional amine group to block self ligation of splint.
- **Figure 3:**: **Reaction scheme of a Labeling method of the invention.** Shown is an embodiment where the oligonucleotide probe is labelled at the 3 prime end.
- **Figure 4:**: **Gapdh mRNA smFISH detection in Hepa** 1-6. Blue staining is DAPI staining for nuclei and white dots are smFISH signal for Gapdh under epifluorescence microscope.
- **Figure 5:**: **Gapdh mRNA smFISH detection in Hepa** 1-6. Blue staining is DAPI staining for nuclei and color dots are smFISH signal for Gapdh at difference z-axis.
- **Figure 6:**: **Gapdh mRNA smFISH dual color detection in mouse** NSCs. Blue staining is DAPI staining for nuclei and green or red color dots are smFISH signal for Gapdh.

In the Sequences:
SEQ ID NO:1 label-carrier-oligonucleotide cta aat cca tta-*ATTO550*
SEQ ID NO:2 complementary-splint-oligonucleotide ATGGATTTAGNNNN-NH2
SEQ ID NO:3-34 mouse Gapdh mRNA coding region specific probe.

| | |
|---|---|
| Seq No.3 | ATGAACCTAAGCTGGGA |
| Seq No.4 | AGGAAACACTCTCCTGA |
| Seq No.5 | TCACCATTTTGTCTACG |
| Seq No.6 | CCAAATCCGTTCACACC |
| Seq No.7 | TCTCCACTTTGCCACTG |
| Seq No.8 | AAGGGGTCGTTGATGGCAA |
| Seq No.9 | GACCATGTAGTTGAGGT |
| Seq No.10 | TGGAGTCATACTGGAAC |
| Seq No.11 | GTGCCGTTGAATTTGCC |
| Seq No.12 | CTTCCCATTCTCGGCCTTGA |
| Seq No.13 | AAGATGGTGATGGGCTT |
| Seq No.14 | ATGTTAGTGGGGTCTCG |
| Seq No.15 | ACGACATACTCAGCACC |
| Seq No.16 | CCATGGTGGTGAAGACA |
| Seq No.17 | AGATGATGACCCTTTTG |
| Seq No.18 | ACAAACATGGGGGCATC |
| Seq No.19 | GACAATCTTGAGTGAGT |
| Seq No.20 | AGTTGGTGGTGCAGGAT |
| Seq No.21 | CCAAAGTTGTCATGGAT |
| Seq No.22 | GGTCATGAGCCCTTCCACAA |
| Seq No.23 | TCTTCTGGGTGGCAGTGAT |
| Seq No.24 | ATGATGTTCTGGGCAGC |
| Seq No.25 | AGTGAGCTTCCCGTTCA |
| Seq No.26 | TAGGAACACGGAAGGCCAT |
| Seq No.27 | ACTTGGCAGGTTTCTCC |
| Seq No.28 | ACCACCTTCTTGATGTC |
| Seq No.29 | AAGATGCCCTTCAGTGG |
| Seq No.30 | GTTGAAGTCGCAGGAGA |
| Seq No.31 | AGGTGGAAGAGTGGGAGTT |
| Seq No.32 | TTGAGAGCAATGCCAGC |
| Seq No.33 | GGAAATGAGCTTGACAA |
| Seq No.34 | AGCCGTATTCATTGTCA |

SEQ ID NO: 35 label-carrier-oligonucleotide for dual color
CTAAA**z**CCATACGGCGCAACT-*ATTO550*, z for FAM-dT.

### EXAMPLES

In order to make smFISH probes easily accessible and producible in any molecular biology and clinical laboratory in the world, the inventors started a journey to re-invent current smFISH probe library preparation to render the method to easier and cheaper. Instead of using EDC based chemistry typically used for commercial probes, the inventors thought using an enzymatic labelling approach to generate a smFISH probe library. This approach was thought to be cheaper and easier to set up in a laboratory. By this critical change, the starting probe library can be synthesized without a terminal amine group on each oligo in the library, which directly reduces the price of single oligo from 15 euro to 2.5 euro (Table 1). This is a huge reduction in the cost of smFISH probe making. For a typical smFISH probe library, there has to be minimally 32, and up to 96 oligos.

**Table 1: Price comparison for various smFISH probes**

| Method | Price (Euro, Sigma) | Probe (nmol) | Reactions (12.5 pmol/reaction) | Price/Reaction (Euro) |
|---|---|---|---|---|
| Stellaris FISH (Biosearch) | 646 | 5 | 400 | 1.6 |
| smFISH (Raj 2008) | 14.85 × 32 = 475.2 | 15 × 32 × 0.2 = 96 | 7680 | 0.06 |
| RainbowFISH (Haikun Liu's lab) | 2.55 × 32 = 81.6 | 15 × 32 × 0.2 = 96 | 7680 | 0.01 |

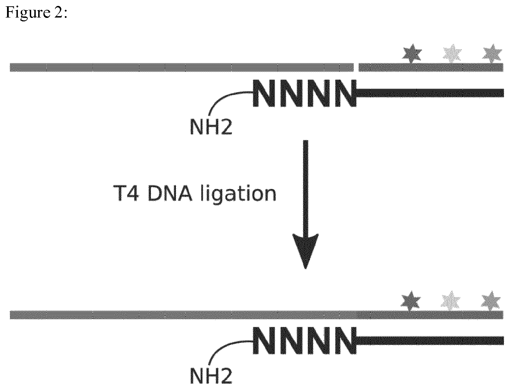

Several enzymes could be potentially used to label a ssDNA oligo in a smFISH probe library with a fluorescently labelled oligo. One possibility is to use T4 DNA ligase, which was here chosen because of the high efficiency and low cost of this enzyme. The inventors have also generated an amine-blocked splint for DNA ligation, which will not generate a self-ligation product from the splint oligonucleotide (Figure 2).

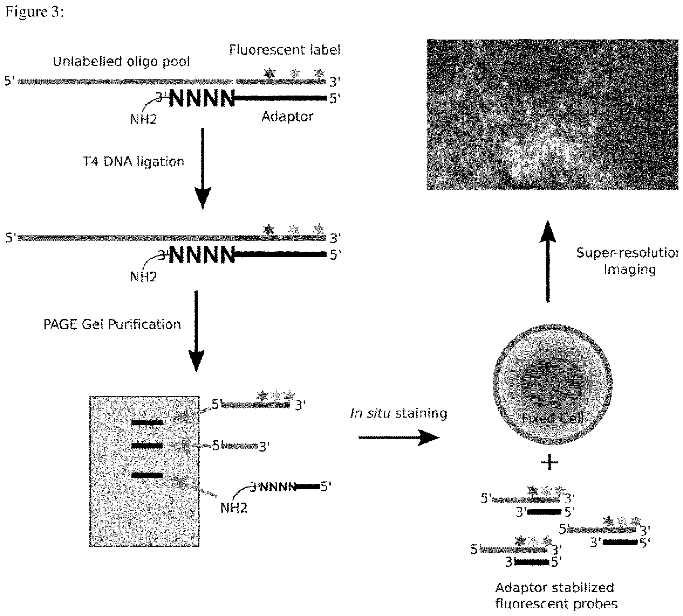

A complete sketch of one example of a labelling process is provided in figure 3. In one alternative example the the label carrying oligonucleotide is ligated to the probe oligo at the 5 prime end. In this example also the arrangement of the splint oligo is reversed.

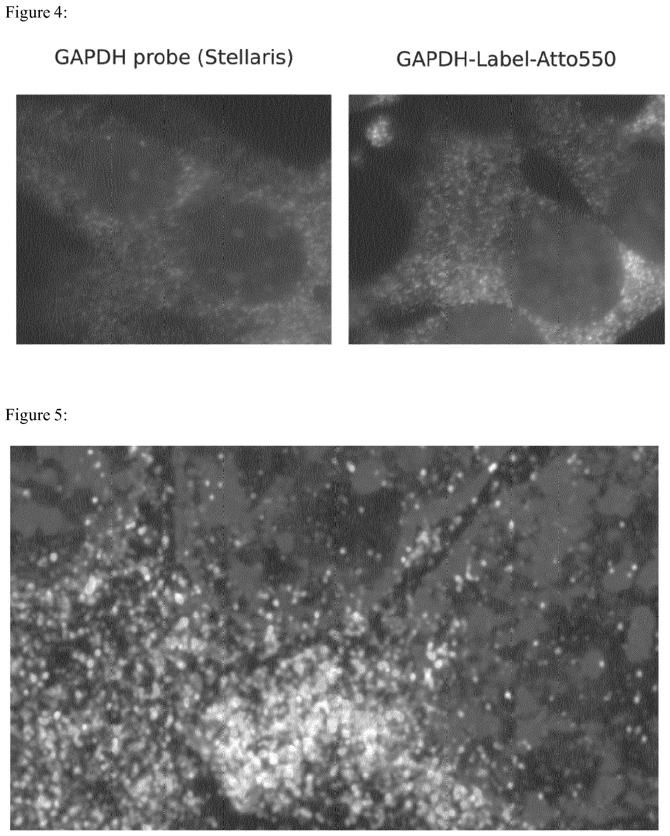

After careful purification on the preparative Urea-PAGE gel, highly purified smFISH probe library for Gapdh was obtained, as well as Bdnf and other two working smFISH probe libraries. One example of Gapdh mRNA staining in mouse Hepa 1-6 cell line, in comparison to commercially available probe for Gapdh, is shown in Figure 4. With a more advanced imaging method from Zeiss, the inventors were able to acquire better smFISH images across deep tissue using airyscan® (Figure 5).

With a similar labelling strategy, the inventors have successfully conjugated smFISH probes with multiple fluorophores at once, which constitutes another example of the present invention. In the following a multiple label probe according to the invention is referred to as "RainbowFISH". In this embodiment of the invention it is critical to include a complementary DNA oligo to form duplex for the common label sequence with fluorophore carrying labelling oligonucleotide. Such a duplex forming complementary oligo is referred to as "adaptor" oligonucleotide. Without this adaptor oligo, the multiple colored smFISH probe will have strong fluorescent quenching.

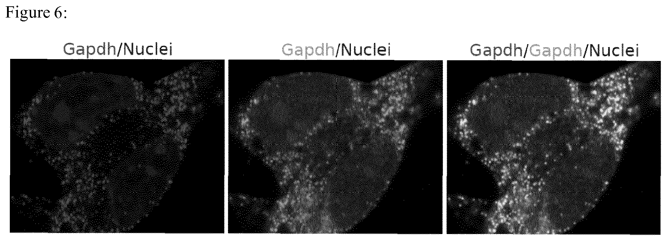

Further shown is one example for Gapdh mRNA detection in mouse neuronal stem cell with a two color RainbowFISH (Figure 6). The Gapdh single molecular mRNA dots are all perfectly colocalized with the green and the red signal. This not only increases the specificity for Gapdh mRNA detection *in situ,* but also allows a multiple color combination to barcode each mRNA species in one detection hybridization.

The multiple color labelling of smFISH probe would also allow for brighter spots for the smFISH image if single fluorophores are not strong enough. Also the use of multiple fluorophores increases the signal for each probe in the library, which therefore may reduce the number of probes contained in each library (from minimally 24 to even 5-6 of total probes).

Theoretically, the number of unique combinations is equal to (n+m-1)!/n!(m-1)! (n for number of fluorophores, and m for number of colors of fluorophores). Then just 5 fluorophores at 6 positions will generate 210 unique combination of colors, which translate into 210 different mRNAs in situ detection at single molecular level. If we could apply multiple rounds of hybridization with multiple color probes, it would be easy to finish whole transcriptome in situ imaging and digital quantification in single cell based on similar strategy used in MERFISH.

A general labelling and probe hybridization example is shown below, divided into several major steps:

### 1. Reaction for smFISH probe labelling.

Reaction example for 100 µl (if larger or smaller scaled needed, change accordingly):

| | |
|---|---|
| 3 µl | probe-sequence-oligonucleotide mix (300 pmol) 100uM mix |
| 4.5 µl | label-carrier-oligonucleotide (450 pmol) 100 uM mix |
| 3 µl | complementary-splint-oligonucleotide 1000uM (3nmol) |
| 10 µl | 10 x T4 DNA ligase buffer (NEB, aliquoted before) |
| 50 µl | PEG8000 (50% w/v) |
| 28 µl | H2O |
| 1.5 µl | t4 dna ligase (conc. 2000 U/ul, NEB) |

Then the composition is mixed well and incubated on a PCR machine or temperature controlled water bath at 16 °C for 1 h. The reaction is quenched by adding 900 µl n-butanol to precipitate all oligonucleotides and resolubilize the oligo pellet in 50 µl 90% formide with loading dye (xylene blue + bromophenol blue). The resolubilized oligos are denature at 95 °C for 5 min, and immediately put on ice.

### 2. Gel purification

Meanwhile, prepare UREA PAGE gel with preparative comb (1.5 mm thick, 1 well, 5 well, etc.) for maximally 500 ul sample / gel:

| | |
|---|---|
| 7.2 g | Urea |
| 1.5 ml | 10 x TBE |
| 7.5 ml | 30% acrylamide:bisacrylamide mix (19:1) |
| 75 ul | 10% APS |
| 7.5 ul | TEMED |

Polymerize the gel at RT for 15 min (before remove the comb, check polymerization between wells, slower in glass plate than that in falcon tube). Then the gel needs to be pre-run for 30 min at 300V (current c.a. 30-50mA) in 1 x TBE. Before loading sample, wash each well before loading, and load maximally 150 ul / well in 5 well gel, or 600-700 µl for 1 well gel. Running condition is at 300 V, 30-45 min to separate until xylene blue band migrate between 1/3 and 1/2 of the gel. The gel can be removed from the plates and cut to get the thin band of fluorescently labelled ligation product of the probe-sequence-oligonucleotide and the label-carrier-oligonucleotide. The band is visible under ambient light and the time to cut the gel should be minimized. The gel piece is homogenized using a microtube pestle inside a 1.5 ml tube and re-suspended in at least 1 ml TE pH8.5 buffer (or 5 vol of gel pieces). Snap freezing in liquid nitrogen/dry ice is required to help further break-down of the gel powder. Afterwards, thaw at 90°C for 5 min before putting the reaction on a rotator overnight at RT. The tube needs to be protected from light, for example with aluminium foil.

### 3. Oligo cleaning and concentration

Removing gel from the TE solution is done by filtering through a 0.22 µm filter tube. The centrifuge at maximally 16000g for 30 min at RT. The collected solution will be concentrated again by adding a total of 6-8 volumes of n-butanol sequentially (optional 3 steps). Then remove the upper phase of butanol maximally. Then add 100 µg glycogen into the solution and precipitate with 6 volumes of cold acetone and wash with 70% ethanol once. The dry pellet will be resolubilized in 30 µl water and concentration is checked using for example a nanodrop machine.

### 4. In situ smFISH staining and imaging

Cell is fixed on coverglass with 3.7% FA for 10 min at RT. Then wash with PBS twice and store in 70% EtOH O/N. Before the *in situ* hybridization, wash twice with 2 x SSC 10% Formamide, 0.1% Tween 20, each time 10 min. Then hybridize in smFISH hybridization 1 x buffer (2xSSC buffer, 10% dextran sulphate, 10% foramide, 1 mg/ml tRNA, 2 mM RVC (Ribonucleoside Vanadyl Complex), 0.2 mg/ml BSA) at 30°C O/N, probe dilution 1:100 (if probe concentration ∼100ng/ul). After hybridization, wash twice with 2 x SSC 10% Formamide, 0.1% Tween 20, each time 30 min, second washing include DAPI staining for nuclei if needed. Mounting sample in ProLong mounting medium from life technologies inc. Imaging smFISH dots can be done on normal wide-field fluorescent microscopy or super-resolution microscopy, including structure-illumination microcopy, STED microscopy etc..

## Claims

1. A method for producing a labeled, or otherwise modified, oligonucleotide probe, the method comprising the steps of:
a. Providing a probe-sequence-oligonucleotide comprising a nucleotide sequence complementary to a target nucleic acid,
b. Providing a label-carrier-oligonucleotide comprising at least one labeling moiety, or other functional moiety, wherein the label-carrier-oligonucleotide has a predetermined nucleotide sequence,
c. Providing a complementary-splint-oligonucleotide, comprising (i) a reverse complementary region having a sequence that is reverse complementary to a sequence of the label-carrier-oligonucleotide and (ii) a random sequence region comprising a random nucleotide sequence;
d. Bringing into contact under hybridizing conditions the probe-sequence-oligonucleotide, label-carrier-oligonucleotide and the complementary-splint-oligonucleotide, to form a complex, wherein, in the complex, a free (unblocked) -OH group is in close spatial proximity to a free (unblocked) phosphate group,
e. Reacting the complex to form a covalent bond between the 3'-OH group and the 5'-phosphate group using a ligase under ligating conditions, to form the labeled, or otherwise modified, oligonucleotide probe,
f. Optionally, removing the complementary-splint-oligonucleotide.

2. A method for producing a labeled, or otherwise modified, oligonucleotide probe according to claim 1, the method comprising the steps of:
a. Providing a probe-sequence-oligonucleotide comprising a nucleotide sequence complementary to a target nucleic acid and having a free (unblocked) -OH group at the 3' end,
b. Providing a label-carrier-oligonucleotide comprising at least one labeling moiety, or other functional moiety, wherein the label-carrier-oligonucleotide has a predetermined nucleotide sequence, and a free (unblocked) phosphate group at the 5' end,
c. Providing a complementary-splint-oligonucleotide, comprising a sequence having in 5' to 3'-direction (i) a reverse complementary region comprising a sequence that is reverse complementary to a sequence of the label-carrier-oligonucleotide; and (ii) a random sequence region comprising a random nucleotide sequence, optionally wherein the complementary-splint-oligonucleotide comprises a 3'-end blocking group,
d. Bringing into contact under hybridizing conditions the probe-sequence-oligonucleotide, label-carrier-oligonucleotide and the complementary-splint-oligonucleotide to form a complex, wherein in the complex the free (unblocked) -OH group at the 3' end of the probe-sequence-oligonucleotide is in close spatial proximity to the free (unblocked) phosphate group at the 5' end of the label-carrier-oligonucleotide,
e. Reacting the complex to form a covalent bond between the 3'-end of the probe-sequence-oligonucleotide and the 5'-end of the label-carrier-oligonucleotide using a ligase under ligating conditions, to form the labeled, or otherwise modified, oligonucleotide probe,
f. Optionally, removing the complementary-splint-oligonucleotide.

3. A method for producing a labeled, or otherwise modified, oligonucleotide probe, the method comprising the steps of:
a. Providing a probe-sequence-oligonucleotide comprising a nucleotide sequence complementary to a target nucleic acid, having a free (unblocked) -phosphate group at the 5' end,
b. Providing a label-carrier-oligonucleotide comprising at least one labeling moiety, or other functional moiety, wherein the label-carrier-oligonucleotide has a predetermined nucleotide sequence, and a free (unblocked) -OH group at the 3' end,
c. Providing a complementary-splint-oligonucleotide, comprising a sequence having in 3' to 5'-direction (i) a reverse complementary region comprising a sequence that is reverse complementary to the sequence of the label-carrier-oligonucleotide; and (ii) a random sequence region comprising a random nucleotide sequence, and wherein the complementary-splint-oligonucleotide comprises a 3'-end blocking group,
d. Bringing into contact under hybridizing conditions the probe-sequence-oligonucleotide, label-carrier-oligonucleotide and the complementary-splint-oligonucleotide to form a complex, wherein in the complex the free (unblocked) -OH group at the 3' end of the label-carrier-oligonucleotide is in close spatial proximity to the free (unblocked) phosphate group at the 5' end of the probe-sequence-oligonucleotide,
e. Forming a covalent bond between the 5'-end of the probe-sequence-oligonucleotide and the 3'-end of the label-carrier-oligonucleotide using a ligase under ligating conditions, to form the labeled, or otherwise modified, oligonucleotide probe,
f. Optionally, removing the complementary-splint-oligonucleotide.

4. The method according to any one of the preceding claims, wherein the label-carrier-oligonucleotide comprises two or more, preferably three, four or five, labeling moieties.

5. The method according to claim 4, wherein the two or more, preferably three, four or five, labeling moieties, comprise at least two or more different labeling moieties, or other functional moieties.

6. The method according to any one of the preceding claims, comprising purifying the ligated product of the probe-sequence-oligonucleotide and label-carrier-oligonucleotide.

7. The method according to any one of claims 1 to 6, comprising a further step of providing an adaptor oligonucleotide comprising a sequence complementary to a sequence of the label-carrier-oligonucleotide, and bringing into contact under hybridizing conditions the ligated product of the probe-sequence-oligonucleotide and label-carrier-oligonucleotide with the adaptor oligonucleotide, to form a stabilized oligonucleotide probe.

8. The method according to any one of the preceding claims, wherein the probe-sequence-oligonucleotide has a length of between 20 to 300 nucleotides.

9. The method according to any one of the preceding claims, wherein the random sequence region of the complementary-splint-oligonucleotide consists of 2 to 10, preferably 2 to 8, more preferably 2 to 6, and most preferably 4 nucleotides.

10. A method for generating a single molecule Fluorescent In-Situ Hybridization (smFISH) probe library, comprising producing at least two fluorescent labeled oligonucleotide probes according to a method of any one of claims 1 to 9, wherein the at least two fluorescent oligonucleotide probes are capable of binding to one target nucleic acid.

11. The method according to claim 10, wherein the at least two fluorescent labeled oligonucleotide probes are at least 10, preferably at least 30, more preferably 30 to 150, and most preferably about 100 fluorescent labeled oligonucleotide probes, and wherein each of said fluorescent labeled oligonucleotide probes is capable of binding to the one target nucleic acid.

12. A labeling kit for labeling a oligonucleotide probe, the kit comprising a label-carrier-oligonucleotide comprising at least one labeling moiety, or other functional moiety, wherein the label-carrier-oligonucleotide has a predetermined nucleotide sequence; a complementary-splint-oligonucleotide, comprising (i) a reverse complementary region having a sequence that is reverse complementary to a sequence of the label-carrier-oligonucleotide and (ii) a random sequence region comprising a degenerated nucleotide sequence.

## Patentansprüche

1. Ein Verfahren zum Herstellen einer markierten oder anderweitig modifizierten Oligonukleotidsonde, wobei das Verfahren die folgenden Schritte umfasst:
a. Bereitstellen eines Sondensequenzoligonukleotids, das eine Nukleotidsequenz umfasst, die zu einer Zielnukleinsäure komplementär ist,
b. Bereitstellen eines Markierungsträgeroligonukleotids, das mindestens eine Markiereinheit oder eine andere funktionelle Einheit umfasst, wobei das Markierungsträgeroligonukleotid eine zuvor bestimmte Nukleotidsequenz aufweist,
c. Bereitstellen eines Komplementärschienenoligonukleotids, das Folgendes umfasst: (i) einen umgekehrt komplementären Bereich, der eine Sequenz aufweist, die zu einer Sequenz des Markierungsträgeroligonukleotids umgekehrt komplementär ist, und (ii) einen zufälligen Sequenzbereich, der eine zufällige Nukleotidsequenz umfasst;
d. In Kontakt bringen des Sondensequenzoligonukleotids, des Markierungsträgeroligonukleotids und des Komplementärschienenoligonukleotids unter Hybridisierungsbedingungen, um einen Komplex auszubilden, wobei sich in dem Komplex eine freie (nicht blockierte) -OH-Gruppe in enger räumlicher Nähe zu einer freien (nicht blockierten) Phosphatgruppe befindet,
e. Umsetzen des Komplexes, um eine kovalente Bindung zwischen der 3'-OH-Gruppe und der 5'-Phosphatgruppe unter Verwendung einer Ligase unter Ligationsbedingungen auszubilden, um die markierte oder auf andere Weise modifizierte Oligonukleotidsonde auszubilden,
f. optional, Entfernen des Komplementärschienenoligonukleotids.

2. Ein Verfahren zum Herstellen einer markierten oder anderweitig modifizierten Oligonukleotidsonde nach Anspruch 1, wobei das Verfahren die folgenden Schritte umfasst:
a. Bereitstellen eines Sondensequenzoligonukleotids, das eine Nukleotidsequenz umfasst, die zu einer Zielnukleinsäure komplementär ist, und eine freie (nicht blockierte) -OH-Gruppe an dem 3'-Ende aufweist,
b. Bereitstellen eines Markierungsträgeroligonukleotids, das mindestens eine Markiereinheit oder eine andere funktionelle Einheit umfasst, wobei das Markierungsträgeroligonukleotid eine zuvor bestimmte Nukleotidsequenz und eine freie (nicht blockierte) Phosphatgruppe an dem 5'-Ende aufweist,
c. Bereitstellen eines Komplementärschienenoligonukleotids, das eine Sequenz umfasst, die Folgendes aufweist: in 5'- bis 3'-Richtung (i) einen umgekehrt komplementären Bereich, der eine Sequenz umfasst, die zu einer Sequenz des Markierungsträgeroligonukleotids umgekehrt komplementär ist; und (ii) einen zufälligen Sequenzbereich, der eine zufällige Nukleotidsequenz umfasst, optional wobei das Komplementärschienenoligonukleotid eine Blockierungsgruppe an dem 3'-Ende umfasst,
d. In Kontakt bringen des Sondensequenzoligonukleotids, des Markierungsträgeroligonukleotids und des Komplementärschienenoligonukleotids unter Hybridisierungsbedingungen, um einen Komplex auszubilden, wobei sich in dem Komplex die freie (nicht blockierte) -OH-Gruppe an dem 3'-Ende in enger räumlicher Nähe zu der freien (nicht blockierten) Phosphatgruppe an dem 5'-Ende des Markierungsträgeroligonukleotids befindet,
e. Umsetzen des Komplexes, um eine kovalente Bindung zwischen dem 3'-Ende des Sondensequenzoligonukleotids und dem 5'-Ende des Markierungsträgeroligonukleotids unter Verwendung einer Ligase unter Ligationsbedingungen auszubilden, um die markierte oder auf andere Weise modifizierte Oligonukleotidsonde auszubilden,
f. optional, Entfernen des Komplementärschienenoligonukleotids.

3. Ein Verfahren zum Herstellen einer markierten oder anderweitig modifizierten Oligonukleotidsonde, wobei das Verfahren die folgenden Schritte umfasst:
a. Bereitstellen eines Sondensequenzoligonukleotids, das eine Nukleotidsequenz umfasst, die zu einer Zielnukleinsäure komplementär ist, das eine freie (nicht blockierte) Phosphatgruppe an dem 5'-Ende aufweist,
b. Bereitstellen eines Markierungsträgeroligonukleotids, das mindestens eine Markiereinheit oder eine andere funktionelle Einheit umfasst, wobei das Markierungsträgeroligonukleotid eine zuvor bestimmte Nukleotidsequenz und eine freie (nicht blockierte) Phosphatgruppe an dem 3'-Ende aufweist,
c. Bereitstellen eines Komplementärschienenoligonukleotids, das eine Sequenz umfasst, die Folgendes aufweist: in 3'- bis 5'-Richtung (i) einen umgekehrt komplementären Bereich, der eine Sequenz umfasst, die zu der Sequenz des Markierungsträgeroligonukleotids umgekehrt komplementär ist; und (ii) einen zufälligen Sequenzbereich, der eine zufällige Nukleotidsequenz umfasst, und wobei das Komplementärschienenoligonukleotid eine Blockierungsgruppe an dem 3'-Ende umfasst,
d. In Kontakt bringen des Sondensequenzoligonukleotids, des Markierungsträgeroligonukleotids und des Komplementärschienenoligonukleotids unter Hybridisierungsbedingungen, um einen Komplex auszubilden, wobei sich in dem Komplex die freie (nicht blockierte) -OH-Gruppe an dem 3'-Ende des Markierungsträgeroligonukleotids in enger räumlicher Nähe zu der freien (nicht blockierten) Phosphatgruppe an dem 5'-Ende des Sondensequenzoligonukleotids befindet,
e. Ausbilden einer kovalenten Bindung zwischen dem 5'-Ende des Sondensequenzoligonukleotids und dem 3'-Ende des Markierungsträgeroligonukleotids unter Verwendung einer Ligase unter Ligationsbedingungen, um die markierte oder auf andere Weise modifizierte Oligonukleotidsonde auszubilden,
f. optional, Entfernen des Komplementärschienenoligonukleotids.

4. Das Verfahren nach einem der vorhergehenden Ansprüche, wobei das Markierungsträgeroligonukleotid zwei oder mehr, bevorzugt drei, vier oder fünf Markiereinheiten umfasst.

5. Das Verfahren nach Anspruch 4, wobei die zwei oder mehr, bevorzugt drei, vier oder fünf Markiereinheiten mindestens zwei oder mehr verschiedene Markiereinheiten oder andere funktionelle Einheiten umfassen.

6. Das Verfahren nach einem der vorhergehenden Ansprüche, das ein Reinigen des ligierten Produkts des Sondensequenzoligonukleotids und des Markierungsträgeroligonukleotids umfasst.

7. Das Verfahren nach einem der Ansprüche 1 bis 6, das einen weiteren Schritt umfasst: Bereitstellen eines Adaptoroligonukleotids, das eine Sequenz umfasst, die zu einer Sequenz des Markierungsträgeroligonukleotids komplementär ist, und in Kontakt bringen des ligierten Produkts des Sondensequenzoligonukleotids des Markierungsträgeroligonukleotids mit dem Adaptoroligonukleotid unter Hybridisierungsbedingungen, um eine stabilisierte Oligonukleotidsonde auszubilden.

8. Das Verfahren nach einem der vorhergehenden Ansprüche, wobei das Sondensequenzoligonukleotid eine Länge zwischen 20 und 300 Nukleotiden aufweist.

9. Das Verfahren nach einem der vorhergehenden Ansprüche, wobei der zufällige Sequenzbereich des Komplementärschienenoligonukleotids aus 2 bis 10, bevorzugt 2 bis 8, stärker bevorzugt 2 bis 6 und am stärksten bevorzugt 4 Nukleotiden besteht.

10. Ein Verfahren zum Erzeugen einer Sondenbibliothek einer Einzelmolekül-Fluoreszenzin-situ-Hybridisierung (*single molecule fluorescent in-situ hybridization* - smFISH), die das Herstellen von mindestens zwei fluoreszierenden markierten Oligonukleotidsonden gemäß einem Verfahren nach einem der Ansprüche 1 bis 9 umfasst, wobei die mindestens zwei fluoreszierenden Oligonukleotidsonden in der Lage sind, sich an eine Zielnukleinsäure zu binden.

11. Das Verfahren nach Anspruch 10, wobei die mindestens zwei fluoreszierenden markierten Oligonukleotidsonden mindestens 10, bevorzugt mindestens 30, stärker bevorzugt 30 bis 150 und am stärksten bevorzugt etwa 100 fluoreszierende markierte Oligonukleotidsonden sind, und wobei jede der fluoreszierenden markierten Oligonukleotidsonden in der Lage ist, sich an die eine Zielnukleinsäure zu binden.

12. Ein Markier-Kit zum Markieren einer Oligonukleotidsonde, wobei das Kit Folgendes umfasst: ein Markierungsträgeroligonukleotid, das mindestens eine Markiereinheit oder eine andere funktionelle Einheit umfasst, wobei das Markierungsträgeroligonukleotid eine zuvor bestimmte Nukleotidsequenz aufweist; ein Komplementärschienenoligonukleotid, das Folgendes umfasst (i) einen umgekehrt komplementären Bereich, der eine Sequenz aufweist, die zu einer Sequenz des Markierungsträgeroligonukleotids umgekehrt komplementär ist, und (ii) einen zufälligen Sequenzbereich, der eine degenerierte Nukleotidsequenz umfasst.

## Revendications

1. Procédé de production d'une sonde oligonucléotidique marquée, ou modifiée d'une autre manière, le procédé comprenant les étapes consistant à :
a. Fournir un oligonucléotide à séquence-sonde comprenant une séquence nucléotidique complémentaire à un acide nucléique cible,
b. Fournir un oligonucléotide porteur de marqueur comprenant au moins une fraction de marquage ou autre fraction fonctionnelle, l'oligonucléotide porteur de marqueur possédant une séquence nucléotidique prédéterminée,
c. Fournir un oligonucléotide d'attelle complémentaire, comprenant (i) une région inversement complémentaire possédant une séquence qui est en complémentarité inverse avec une séquence de l'oligonucléotide porteur de marqueur et (ii) une région à séquence aléatoire comprenant une séquence nucléotidique aléatoire,
d. Mettre en contact, dans des conditions d'hybridation, l'oligonucléotide à séquence-sonde, l'oligonucléotide porteur de marqueur et l'oligonucléotide attelle complémentaire, pour former un complexe, dans le complexe, un groupement -OH libre (non bloqué) étant très proche spatialement d'un groupement phosphate libre (non bloqué),
e. Faire réagir le complexe pour former une liaison covalente entre le groupement 3'-OH et le groupement 5'-phosphate à l'aide d'une ligase dans des conditions de ligature, pour former la sonde oligonucléotidique marquée ou modifiée d'une autre manière,
f. Éventuellement, éliminer l'oligonucléotide d'attelle complémentaire.

2. Procédé de production d'une sonde oligonucléotidique marquée ou modifiée d'une autre manière selon la revendication 1, le procédé comprenant les étapes consistant à :
a. Fournir un oligonucléotide à séquence-sonde comprenant une séquence nucléotidique complémentaire à un acide nucléique cible et possédant un groupement -OH libre (non bloqué) à l'extrémité 3',
b. Fournir un oligonucléotide porteur de marqueur comprenant au moins une fraction de marquage ou autre fraction fonctionnelle, l'oligonucléotide porteur de marqueur possédant une séquence nucléotidique prédéterminée, et un groupement phosphate libre (non bloqué) à l'extrémité 5',
c. Fournir un oligonucléotide d'attelle complémentaire, comprenant une séquence possédant dans le sens 5' à 3' (i) une région inversement complémentaire comprenant une séquence qui est inversement complémentaire d'une séquence de l'oligonucléotide porteur de marqueur ; et (ii) une région à séquence aléatoire comprenant une séquence nucléotidique aléatoire, l'oligonucléotide d'attelle complémentaire comprenant éventuellement un groupement bloquant l'extrémité 3',
d. Mettre en contact, dans des conditions d'hybridation, l'oligonucléotide à séquence-sonde, l'oligonucléotide porteur de marqueur et l'oligonucléotide d'attelle complémentaire pour former un complexe, le groupement -OH libre (non bloqué) à l'extrémité 3' de l'oligonucléotide à séquence-sonde étant très proche spatialement du groupement phosphate libre (non bloqué) à l'extrémité 5' de l'oligonucléotide porteur de marqueur,
e. Faire réagir le complexe pour former une liaison covalente entre l'extrémité 3' de l'oligonucléotide à séquence-sonde et l'extrémité 5' de l'oligonucléotide porteur de marqueur à l'aide d'une ligase dans des conditions de ligature, pour former la sonde oligonucléotidique marquée ou modifiée d'une autre manière,
f. Éventuellement, éliminer l'oligonucléotide d'attelle complémentaire.

3. Procédé de production d'une sonde oligonucléotidique marquée, ou modifiée d'une autre manière, le procédé comprenant les étapes consistant à :
a. Fournir un oligonucléotide à séquence-sonde comprenant une séquence nucléotidique complémentaire à un acide nucléique cible, possédant un groupement phosphate libre (non bloqué) à l'extrémité 5',
b. Fournir un oligonucléotide porteur de marqueur comprenant au moins une fraction de marquage ou autre fraction fonctionnelle, l'oligonucléotide porteur de marqueur possédant une séquence nucléotidique prédéterminée, et un groupement -OH libre (non bloqué) à l'extrémité 3',
c. Fournir un oligonucléotide d'attelle complémentaire, comprenant une séquence possédant dans le sens 3' à 5' (i) une région inversement complémentaire comprenant une séquence qui est inversement complémentaire à la séquence de l'oligonucléotide porteur de marqueur ; et (ii) une région à séquence aléatoire comprenant une séquence nucléotidique aléatoire, et l'oligonucléotide d'attelle complémentaire comprenant un groupement bloquant à l'extrémité 3',
d. Mettre en contact, dans des conditions d'hybridation, l'oligonucléotide à séquence-sonde, l'oligonucléotide porteur de marqueur et l'oligonucléotide attelle complémentaire pour former un complexe, dans lequel dans le complexe le groupement -OH libre (non bloqué) à l'extrémité 3' de l'oligonucléotide porteur de marqueur est très proche spatialement du groupement phosphate libre (non bloqué) à l'extrémité 5' de l'oligonucléotide à séquence-sonde,
e. Former une liaison covalente entre l'extrémité 5' de l'oligonucléotide à séquence-sonde et l'extrémité 3' de l'oligonucléotide porteur de marqueur à l'aide d'une ligase dans des conditions de ligature, pour former la sonde oligonucléotidique marquée ou modifiée d'une autre manière,
f. Éventuellement, éliminer l'oligonucléotide d'attelle complémentaire.

4. Procédé selon l'une quelconque des revendications précédentes, l'oligonucléotide porteur de marqueur comprenant deux ou plus, de préférence trois, quatre ou cinq fractions de marquage.

5. Procédé selon la revendication 4, les deux ou plus, de préférence trois, quatre ou cinq fractions de marquage, comprenant au moins deux fractions de marquage ou autres fractions fonctionnelles différentes.

6. Procédé selon l'une quelconque des revendications précédentes, comprenant la purification du produit ligaturé de l'oligonucléotide à séquence-sonde et de l'oligonucléotide porteur de marqueur.

7. Procédé selon l'une quelconque des revendications 1 à 6, comprenant une étape supplémentaire consistant à fournir un oligonucléotide adaptateur comprenant une séquence complémentaire d'une séquence de l'oligonucléotide porteur de marqueur, et mettre en contact dans des conditions d'hybridation le produit ligaturé de l'oligonucléotide à séquence-sonde et de l'oligonucléotide porteur de marqueur avec l'oligonucléotide adaptateur, pour former une sonde oligonucléotidique stabilisée.

8. Procédé selon l'une quelconque des revendications précédentes, l'oligonucléotide à séquence-sonde possédant une longueur comprise entre 20 et 300 nucléotides.

9. Procédé selon l'une quelconque des revendications précédentes, la région à séquence aléatoire de l'oligonucléotide d'attelle complémentaire étant constituée de 2 à 10, de préférence de 2 à 8, plus préférablement de 2 à 6, et le plus préférablement de 4 nucléotides.

10. Procédé de génération d'une bibliothèque de sondes d'hybridation in situ en fluorescence à molécule unique (smFISH), comprenant la production d'au moins deux sondes oligonucléotidiques marquées par fluorescence selon un procédé selon l'une quelconque des revendications 1 à 9, les au moins deux sondes oligonucléotidiques fluorescentes étant capables de se lier à un acide nucléique cible.

11. Procédé selon la revendication 10, les au moins deux sondes oligonucléotidiques marquées par fluorescence représentant au moins 10, de préférence au moins 30, plus préférablement entre 30 et 150, et le plus préférablement environ 100 sondes oligonucléotidiques marquées par fluorescence, et chacune desdites sondes oligonucléotidiques marquées par fluorescence étant capable de se lier à un acide nucléique cible.

12. Kit de marquage pour le marquage d'une sonde oligonucléotidique, le kit comprenant un oligonucléotide porteur de marqueur comprenant au moins une fraction de marquage ou autre fraction fonctionnelle, l'oligonucléotide porteur de marqueur possédant une séquence nucléotidique prédéterminée ; un oligonucléotide d'attelle complémentaire, comprenant (i) une région inversement complémentaire possédant une séquence qui est inversement complémentaire à une séquence de l'oligonucléotide porteur de marqueur et (ii) une région à séquence aléatoire comprenant une séquence nucléotidique dégénérée.


### SEQUENCE LISTING

<110> Deutsches Krebsforschungszentrum Stiftung des oeffentlichen Rechts
<120> METHOD FOR LABELING OLIGONUCLEOTIDE PROBES
<130> D31525WO
<150> EP16190862.9
   <151> 2016-09-27
<160> 35
<170> PatentIn version 3.5
<210> 1
   <211> 12
   <212> DNA
   <213> artificial
<220>
   <223> label-carrier-oligonucleotide
<220>
   <221> modified_base
   <222> (12)..(12)
   <223> ATTO550
<400> 1
   ctaaatccat ta 12
<210> 2
   <211> 14
   <212> DNA
   <213> artificial
<220>
   <223> complementary-splint-oligonucleotide
<220>
   <221> misc_feature
   <222> (11)..(14)
   <223> n is a, c, g, or t
<400> 2
   atggatttag nnnn 14
<210> 3
   <211> 17
   <212> DNA
   <213> Mus musculus
<400> 3
   atgaacctaa gctggga 17
<210> 4
   <211> 17
   <212> DNA
   <213> Mus musculus
<400> 4
   aggaaacact ctcctga 17
<210> 5
   <211> 17
   <212> DNA
   <213> Mus musculus
<400> 5
   tcaccatttt gtctacg 17
<210> 6
   <211> 17
   <212> DNA
   <213> Mus musculus
<400> 6
   ccaaatccgt tcacacc 17
<210> 7
   <211> 17
   <212> DNA
   <213> Mus musculus
<400> 7
   tctccacttt gccactg 17
<210> 8
   <211> 19
   <212> DNA
   <213> Mus musculus
<400> 8
   aaggggtcgt tgatggcaa 19
<210> 9
   <211> 17
   <212> DNA
   <213> Mus musculus
<400> 9
   gaccatgtag ttgaggt 17
<210> 10
   <211> 17
   <212> DNA
   <213> Mus musculus
<400> 10
   tggagtcata ctggaac 17
<210> 11
   <211> 17
   <212> DNA
   <213> Mus musculus
<400> 11
   gtgccgttga atttgcc 17
<210> 12
   <211> 20
   <212> DNA
   <213> Mus musculus
<400> 12
   cttcccattc tcggccttga 20
<210> 13
   <211> 17
   <212> DNA
   <213> Mus musculus
<400> 13
   aagatggtga tgggctt 17
<210> 14
   <211> 17
   <212> DNA
   <213> Mus musculus
<400> 14
   atgttagtgg ggtctcg 17
<210> 15
   <211> 17
   <212> DNA
   <213> Mus musculus
<400> 15
   acgacatact cagcacc 17
<210> 16
   <211> 17
   <212> DNA
   <213> Mus musculus
<400> 16
   ccatggtggt gaagaca 17
<210> 17
   <211> 17
   <212> DNA
   <213> Mus musculus
<400> 17
   agatgatgac ccttttg 17
<210> 18
   <211> 17
   <212> DNA
   <213> Mus musculus
<400> 18
   acaaacatgg gggcatc 17
<210> 19
   <211> 17
   <212> DNA
   <213> Mus musculus
<400> 19
   gacaatcttg agtgagt 17
<210> 20
   <211> 17
   <212> DNA
   <213> Mus musculus
<400> 20
   agttggtggt gcaggat 17
<210> 21
   <211> 17
   <212> DNA
   <213> Mus musculus
<400> 21
   ccaaagttgt catggat 17
<210> 22
   <211> 20
   <212> DNA
   <213> Mus musculus
<400> 22
   ggtcatgagc ccttccacaa 20
<210> 23
   <211> 19
   <212> DNA
   <213> Mus musculus
<400> 23
   tcttctgggt ggcagtgat 19
<210> 24
   <211> 17
   <212> DNA
   <213> Mus musculus
<400> 24
   atgatgttct gggcagc 17
<210> 25
   <211> 17
   <212> DNA
   <213> Mus musculus
<400> 25
   agtgagcttc ccgttca 17
<210> 26
   <211> 19
   <212> DNA
   <213> Mus musculus
<400> 26
   taggaacacg gaaggccat 19
<210> 27
   <211> 17
   <212> DNA
   <213> Mus musculus
<400> 27
   acttggcagg tttctcc 17
<210> 28
   <211> 17
   <212> DNA
   <213> Mus musculus
<400> 28
   accaccttct tgatgtc 17
<210> 29
   <211> 17
   <212> DNA
   <213> Mus musculus
<400> 29
   aagatgccct tcagtgg 17
<210> 30
   <211> 17
   <212> DNA
   <213> Mus musculus
<400> 30
   gttgaagtcg caggaga 17
<210> 31
   <211> 19
   <212> DNA
   <213> Mus musculus
<400> 31
   aggtggaaga gtgggagtt 19
<210> 32
   <211> 17
   <212> DNA
   <213> Mus musculus
<400> 32
   ttgagagcaa tgccagc 17
<210> 33
   <211> 17
   <212> DNA
   <213> Mus musculus
<400> 33
   ggaaatgagc ttgacaa 17
<210> 34
   <211> 17
   <212> DNA
   <213> Mus musculus
<400> 34
   agccgtattc attgtca 17
<210> 35
   <211> 21
   <212> DNA
   <213> artificial
<220>
   <223> label-carrier-oligonucleotide
<220>
   <221> modified_base
   <222> (6)..(6)
   <223> FAM dT
<220>
   <221> modified_base
   <222> (13)..(13)
   <223> FAM dT
<220>
   <221> modified_base
   <222> (21)..(21)
   <223> FAM dT
<400> 35
   ctaaatccat acggcgcaac t 21